# EUROPÄISCHE PATENTANMELDUNG

(11) **EP 3 530 307 A1**
(43) Veröffentlichungstag der Anmeldung: **28.08.2019**
(21) Anmeldenummer: 18185221.1
(22) Anmeldetag: 24.07.2018
(51) Int. Cl.: A61M 5/44

(54) **BEHÄLTERHALTER MIT TEMPERIEREINRICHTUNG FÜR EINEN INJEKTOR**

(30) Priorität: 22.02.2018 DE 102018104002
(71) Anmelder: ulrich GmbH & Co. KG, 89081 Ulm (DE)
(72) Erfinder: Geangu, Anamaria, 89081 Ulm (DE); Götz, Markus, 89134 Blaustein (DE)
(74) Vertreter: Charrier Rapp & Liebau

(57) **Zusammenfassung**

Bei einem Behälterhalter für einen Injektor (I) zur Injektion eines Fluids in den menschlichen oder tierischen Körper ist wenigstens eine Behälteraufnahme (10) zur Aufnahme eines mit dem Fluid gefüllten Behälters (B) und eine Temperiereinrichtung (20) zur Temperierung des Behälter (B) auf eine vorgegebene Temperatur vorgesehen. Um Behälter mit unterschiedlichen Formen und Größen in den Behälterhalter einsetzen und mit der Temperiereinrichtung (20) auf eine gewünschte Temperatur ohne Wärmeverluste temperieren zu können, und um ein einfaches und behinderungsfreies Einsetzen und Herausnehmen des Behälters zu ermöglichen, ist die Temperiereinrichtung (20) an eine Transporteinrichtung (30) gekoppelt, mit der die Temperiereinrichtung (20) mit dem in der Behälteraufnahme (10) eingesetzten Behälter (B) in und außer thermischem Kontakt bringbar ist.

## Beschreibung

Die Erfindung betrifft einen Behälterhalter für einen Injektor zur Injektion von Fluiden, bspw. von Kontrastmitteln, in den menschlichen oder tierischen Körper nach dem Oberbegriff des Anspruchs 1.

In bildgebenden Verfahren am menschlichen oder tierischen Körper zum Zwecke der medizinischen Diagnostik und der Darstellung von Strukturen und Funktionen der Körpergewebe und -Organe, wie z.B. der Magnetresonanztomographie (MRT) und der Computertomographie (CT), werden dem Körper häufig vor oder während der Durchführung des Verfahrens Kontrastmittel verabreicht um den Kontrast der aufgenommenen Bilder zu verbessern. Durch Vergleich der Aufnahmen des bildgebenden Verfahrens ohne und mit Gabe von Kontrastmitteln kann die Aussagekraft der aufgenommenen Bilder verbessert und es können beispielsweise durch eine intensivere Weißfärbung gekennzeichnete Entzündungsherde oder Tumore besser erkannt werden.

Zur intravenösen Verabreichung der Kontrastmittel werden Injektoren verwendet, welche ein in einem Vorratsbehälter bevorratetes Kontrastmittel mittels einer Pumpe in einen Patientenschlauch transferieren, welcher zur intravenösen Verabreichung des Kontrastmittels in den Körper eines Patienten mit einer Kanüle verbunden ist. Zum Transfer des Kontrastmittels von dem Vorratsbehälter in den Patientenschlauch wird regelmäßig eine Fördervorrichtung mit einer Schlauchpumpe verwendet. Dabei wird zwischen dem Vorratsbehälter und dem Patientenschlauch eine Transfereinrichtung in Form eines Schlauchsystems oder einer Kassette mit Fluidkanälen eingesetzt, welche das Kontrastmittel vom Vorratsbehälter in einen Pumpenschlauch überführen, der in die Schlauchpumpe eingelegt und mit dem Patientenschlauch verbunden ist. Die Schlauchpumpe fördert das Kontrastmittel vom Vorratsbehälter in den Patientenschlauch.

Ein solcher Injektor zur Applizierung von Kontrastmitteln für die Röntgen- und Kernspintomographie ist bspw. aus der DE 20 2015 102 187 U1 bekannt. Dieser Injektor umfasst mehrere Vorratsbehälter, die mit einem zu injizierenden Fluid (bspw. ein Kontrastmittel oder eine Spüllösung) gefüllt sind, sowie eine austauschbar am Injektor angeordnete Kassette, die mit einem Pumpenschlauch und über Verbindungsschläuche mit den Vorratsbehältern verbunden ist. Die Kassette umfasst einen Korpus mit darin ausgeformten Fluidkanälen, welche über die Verbindungsschläuche mit den Vorratsbehältern und mit dem Pumpenschlauch in Verbindung stehen. Zur Förderung des zu injizierenden Fluids (Kontrastmittel oder Spüllösung, bspw. NaCl) aus den Vorratsbehältern durch die Fluidkanäle der Kassette, den daran angeschlossenen Pumpenschlauch und in den Patientenschlauch ist eine Schlauchpumpe (Rollenpumpe) vorgesehen, welche den Pumpenschlauch zyklisch gegen ein Gegenlager quetscht und dadurch das sich im Pumpenschlauch befindliche Fluid in den Patientenschlauch fördert.

Fluide, die dem menschlichen oder Tierischen Körper bspw. durch Infusion oder intravenöse Injektion verabreicht werden, sollten zur Vermeidung von Hypothermie, Traumata oder anderer Gesundheitsbeeinträchtigungen zumindest annähernd die Temperatur des Körpers aufweisen. Um das zu injizierende Fluid auf Körpertemperatur zu bringen und zu halten, sind aus dem Stand der Technik Injektoren mit Heizeinrichtungen bekannt, mit denen das Fluid auf Körpertemperatur erwärmt und während des Injektionsvorgangs auf diesem Temperaturniveau gehalten werden kann. So ist bspw. aus der EP 1 526 881 B1 ein Injektor zur Injektion von Fluiden aus einer Spritze in ein tierisches Subjekt bekannt, der über eine beheizbare Spritzenaufnahme zur Aufnahme der mit dem Fluid gefüllten Spritze verfügt. Eine weitere Heizeinrichtung mit einem beheizbaren Fluidschlauch zum Erwärmen von darin geführten Fluiden, die zur Verabreichung in den Körper eines Patienten vorgesehen sind, ist aus der WO 2014/098675 A1 bekannt.

Die in Injektoren integrierten Heizeinrichtungen zum Temperieren von zu injizierenden Fluiden auf Körpertemperatur, wie z.B. die aus der EP 1 526 881 B1 bekannte beheizbare Spritzenaufhahme, weisen den Nachteil auf, dass sie an die Form und Größe des Behälters angepasst sind, in dem sich das Fluid befindet. Es können daher nur Behälter mit einer vorbestimmten Form und Größe verwendet und mit der Heizeinrichtung erwärmt bzw. auf Körpertemperatur gehalten werden. Zu injizierende Fluide wie Kontrastmittel und Spüllösungen sind jedoch in der Regel in Vorratsbehältern mit unterschiedlichen Formen und Größen abgefüllt. Es ist daher nicht möglich, unterschiedliche Vorratsbehälter direkt in eine für einen bestimmten Behälter vorgesehene und entsprechend geformte Aufnahme am Injektor einzusetzen und mit einer Temperiereinrichtung zu erwärmen bzw. auf Körpertemperatur zu halten.

Es besteht daher ein Bedürfnis nach einem Injektor mit einem Behälterhalter, der zur Aufnahme von Behältern mit unterschiedlichen Formen und Größen geeignet und mit einer Temperiereinrichtung ausgestattet ist, mit der unterschiedliche Behälter, die in der Aufnahme eingesetzt sind, erwärmt und insbesondere auf Körpertemperatur gehalten werden können. Die Temperierung soll dabei möglichst energieeffizient ohne Wärmeverluste erfolgen und es soll ein einfaches und behinderungsfreies Einsetzen und Herausnehmen des Behälters ermöglicht werden.

Dieses Bedürfnis wird durch einen Behälterhalter für einen Injektor mit den Merkmalen des Anspruchs 1 erfüllt. Bevorzugte Ausführungsformen des Behälterhalters sind den abhängigen Ansprüchen zu entnehmen. Zur Lösung der Aufgabe trägt ferner ein Verfahren mit den Merkmalen des Anspruchs 15 bei.

Der erfindungsgemäße Behälterhalter umfasst wenigstens eine Behälteraufnahme zur Aufnahme eines mit dem Fluid gefüllten Behälters und eine Temperiereinrichtung zur Temperierung des Behälter auf eine vorgegebene Temperatur, insbesondere die Körpertemperatur eines Patienten, in dessen Körper das Fluid injiziert werden soll. Erfindungsgemäß ist die Temperiereinrichtung an eine Transporteinrichtung gekoppelt, mit der die Temperiereinrichtung mit dem in der Behälteraufnahme eingesetzten Behälter in und außer thermischem Kontakt bringbar ist.

Die Behälteraufnahme ist dabei so ausgebildet, dass unterschiedliche Behälter mit unterschiedlichen Formen und Größen in die wenigstens eine Behälteraufnahme eingesetzt werden können. Mit der Transporteinrichtung kann die Temperiereinrichtung nach dem Einsetzen eines Behälters in die Behälteraufnahme an den eingesetzten Behälter herangeführt werden, um die Temperiereinrichtung unabhängig von der Form und der Größe des Behälters in thermischem Kontakt mit der Außenfläche des Behälters zu bringen. Wenn der Behälter geleert ist, kann die Temperiereinrichtung mittels der Transporteinrichtung wieder von dem eingesetzten Behälter weggeführt werden, so dass sich die Temperiereinrichtung entfernt vom Behälter befindet. Dadurch wird eine einfache Entnahme des geleerten Behälters aus der Behälteraufnahme und ein behinderungsfreies Einsetzen eines neuen, mit einem Injektionsfluid gefüllten Behälter ermöglicht.

Vorteilhaft ist die Temperiereinrichtung mittels der Transporteinrichtung zumindest zwischen einer rückwärtigen Position und mindestens einer vorderen Position verschiebbar, wobei die Temperiereinrichtung in der rückwärtigen Position entfernt von dem in der Behälteraufnahme eingesetzten Behälter ist und in der vorderen Position in thermischem Kontakt mit dem Behälter steht. Um die Transporteinrichtung in der rückwärtigen Position und/oder in der vorderen Position fixieren zu können, ist bevorzugt ein Rastmechanismus vorgesehen. Eine Fixierung der Transporteinrichtung in der rückwärtigen Position ist vorteilhaft, um ein einfaches und behinderungsfreies Einsetzen und Entnehmen eines Behälters in die bzw. aus der Behälteraufnahme zu ermöglichen. Eine Fixierung der Transporteinrichtung in der vorderen Position ist vorteilhaft, um während des Injektionsvorgangs einen guten thermischen Kontakt zwischen der Temperiereinrichtung und dem Behälter zu gewährleisten und beizubehalten.

Da unterschiedliche Behälter mit verschiedenen Formen (bspw. Flaschen, Dosen oder Beutel) und Größen (bspw. mit Füllmengen von 100 ml bis 1000 ml) in die Behälteraufnahme eingesetzt werden sollen, ist es von Vorteil, wenn der Rastmechanismus neben der rückwärtigen Position mehrere vordere Rastpositionen enthält, wobei die vorderen Rastpositionen bevorzugt so ausgewählt sind, dass sich die Temperiereinrichtung in thermischem Kontakt bspw. mit einem Behälter in einer vorgegebenen Standardgröße und -form (bspw. eine 500 ml oder eine 1000 ml-Flasche) befindet, wenn ein entsprechender Behälter in die Behälteraufnahme eingesetzt ist. Der Rastmechanismus kann dabei die Transporteinrichtung in der rückwärtigen Position und/oder in einer der vorderen Rastpositionen fixieren. Es ist jedoch auch möglich, Behälter mit einer nicht standardisierten Größe oder Form in die Behälteraufnahme einzusetzen.

Zum einfachen manuellen Lösen des Rastmechanismus aus der rückwärtigen Position ist zweckmäßig ein manuell betätigbarer Druckknopf vorgesehen, der bei Betätigung eine Verrastung der Transporteinrichtung in der rückwärtigen Position und dadurch eine Bewegung der Temperiereinrichtung in eine vordere Position frei gibt, in der sich die Temperiereinrichtung in thermischem Kontakt mit einem in der Behälteraufnahme eingesetzten Behälter befindet.

In einem Ausführungsbeispiel der Erfindung umfasst die Transporteinrichtung eine Feder und die Temperiereinrichtung wird von der Rückstellkraft der Feder in Richtung eines in der Behälteraufnahme eingesetzten Behälters bewegt, um die Temperiereinrichtung in thermischem Kontakt mit dem Behälter zu bringen. Hierfür kann die Transporteinrichtung ein am Injektor fixierbares Gehäuse und einen gegenüber dem Gehäuse beweglichen Schlitten aufweisen, an dem die Temperiereinrichtung linear verschiebbar angeordnet ist. Der Schlitten ist dabei zweckmäßig in oder an dem Gehäuse verschiebbar an einer Führung gelagert und gegen die Rückstellkraft der Feder entlang der Führung beweglich. Dies ermöglicht es, dass der Schlitten mit der daran befestigten Temperiereinrichtung automatisch von der Feder in Richtung der Behälteraufnahme gedrückt wird, wenn der Rastmechanismus aus der rückwärtigen Position (durch Betätigen des Druckknopfs) gelöst wird. Die Temperiereinrichtung wird dadurch automatisch von der Transporteinrichtung in eine vordere Position zu einen sich in der Behälteraufnahme befindlichen Behälter transportiert, bis die Temperiereinrichtung in (thermischem) Kontakt mit dem Behälter steht. Damit die von der Feder automatisch ausgelöste Bewegung der Temperiereinrichtung nicht zu abrupt oder zu schnell erfolgt, ist die Bewegung des Schlittens bevorzugt durch ein Dämpfungselement gedämpft. Dies kann bspw. durch eine am Schlitten angeordnete Zahnstange realisiert sein, welche mit einem als Rotationsdämpfer ausgebildeten und am Gehäuse der Transporteinrichtung befestigten Dämpfungselement gekoppelt ist. Es ist jedoch auch möglich, das Dämpfungselement am Schlitten und die Zahnstange am Gehäuse zu befestigen. Das Dämpfungselement kann auch als Kolbendämpfer ausgebildet sein. Der Schlitten kann dabei sowohl innerhalb als auch außerhalb, d.h. in oder an dem Gehäuse entlang der Führung verschiebbar gelagert sein. Bevorzugt, aber nicht zwingend, ist der Schlitten von einer Spannkraft in Richtung der Behälteraufnahme vorgespannt. Die Spannkraft kann dabei durch die Feder oder durch andere Vorspannelemente bereit gestellt werden, bspw. durch elastische Bänder.

In der Ausführungsform der Erfindung, in der die Transporteinrichtung eine Feder oder andere Vorspannelemente aufweist und die Temperiereinrichtung von der Rückstellkraft der Feder bzw. der Spannkraft des Vorspannelements in Richtung eines in der Behälteraufnahme eingesetzten Behälters bewegt wird, um die Temperiereinrichtung in thermischem Kontakt mit dem Behälter zu bringen, ist es nicht zwingend erforderlich, dass der Rastmechanismus neben der rückwärtigen Rastposition noch eine oder mehrere vordere Rastpositionen bereitstellt, da die Feder die Temperiereinrichtung ohnehin an die Außenfläche des Behälters andrückt und dadurch einen thermischem Kontakt mit dem Behälter herstellt und während eines Injektionsvorgangs aufrecht erhält. Insbesondere in dieser Ausführungsform der Erfindung können auch Behälter mit einer nicht standardisierten Größe oder Form in die Behälteraufnahme eingesetzt werden.

Nach Beendigung des Injektionsvorgangs oder zum Austausch eines geleerten Behälters gegen einen neuen, mit einem Injektionsfluid gefüllten Behälter kann die Temperiereinrichtung wieder außer Kontakt mit dem Behälter gebracht werden, indem die Temperiereinrichtung mittels der Transporteinrichtung vom Behälter entfernt wird. Hierfür kann zweckmäßig an dem Schlitten ein Griffteil angeformt sein, mit dem der bewegliche Schlitten manuell und entgegen der Rückstellkraft der Feder verschiebbar ist, um ein Zurückschieben des Schlittens aus einer vorderen Position, in der sich die Temperiereinrichtung in thermischem Kontakt mit dem Behälter befindet, in die rückwärtige Position, in der sich die Temperiereinrichtung im Abstand zu dem Behälter befindet, zu ermöglichen. Zweckmäßig wird automatisch die Stromzufuhr für die Temperiereinrichtung abgeschaltet, wenn sich die Temperiereinrichtung nicht im Kontakt mit dem Behälter befindet. Dadurch kann Heizenergie gespart werden.

Um eine möglichst große thermische Kontaktfläche zwischen der Temperiereinrichtung und einem in die Behälteraufnahme eingesetzten Behälter zu erzeugen, ist die Temperiereinrichtung zweckmäßig in dem Bereich einer Wärmeübertragungszone, die in thermischem Kontakt mit der Außenfläche des Behälters kommt, so geformt, dass sich die Temperiereinrichtung unabhängig von der Form und der Größe des Behälters möglichst gut an die Außenfläche des Behälters anschmiegen kann. Dies gewährleistet eine effiziente Wärmeübertragung ohne Wärmeverluste.

Da die herkömmlichen Vorratsbehälter für Kontrastmittel eine zylindrische Form aufweisen, ist es zweckmäßig, wenn zumindest die dem Behälter zugewandte Wärmeübertragungszone der Temperiereinrichtung im Schnitt gebogen bzw. konkav gewölbt ist. Bevorzugt weist die Wärmeübertragungszone der Temperiereinrichtung eine teilzylindrische Form auf, bspw. die Form eines Halb-, Drittel- oder Viertelzylinders.

Um eine effiziente Wärmeübertragung von der Wärmeübertragungszone auf den Behälter zu ermöglichen, ist die Wärmeübertragungszone bevorzugt aus einem elastischen und wärmeleitenden Material. Die Elastizität der Wärmeübertragungszone ermöglicht dabei ein Anschmiegen der Wärmeübertragungszone an die Form des Behälters und somit eine Optimierung der Wärmeübertragungsfläche, wenn die Temperiereinrichtung mittels der Transporteinrichtung mit dem Behälter in Kontakt gebracht worden ist. Zur Erzielung eines optimalen Wärmeübergangs ist die Wärmeübertragungszone von einem Wärmeleitungselement aus einem wärmeleitenden Material, wie z.B. einem Metall, Graphit oder einem wärmeleitfähigen Kunststoff, gebildet, welches an der Außenfläche des Behälters anliegt. Das Wärmeleitungselement kann auch als mehrkomponentiges Kunststoff-Spritzgussteil ausgebildet sein, welches einen Grundkörper aus einem Hartkunststoff und eine elastische Auflage aus einem Elastomer enthält.

Die Temperiereinrichtung enthält bevorzugt ein regelbares Heizelement oder Kühlelement. Als Heizelement können elektrische Heizelemente, bspw. ein mit Strom beaufschlagbarer Heizdraht oder ein PTC-Heizelement, verwendet werden. Ein Peltierelement kann - je nach Richtung des Stromflusses - sowohl als Heiz- als auch als Kühlement eingesetzt werden. Mit dem Heiz- oder Kühlement kann der Behälter und das darin befindliche Fluid auf eine vorgebbare Solltemperatur (insbesondere auf Temperaturen im Bereich von 28 °C bis 37°C) gebracht und/oder während eines Injektionsvorgangs (der einige Minuten bis zu Stunden dauern kann) gehalten werden. Meist werden die mit einem zu injizierenden Fluid gefüllten Behälter vortemperiert und bereits mit einer Temperatur im Bereich einer gewünschten Solltemperatur (insbesondere der Körpertemperatur im Bereich um 37°C) in die Behälteraufnahme eingesetzt. In diesem Fall dient das Heiz- oder Kühlelement zur Aufrechterhaltung der einstellbaren Solltemperatur während des Injektionsvorgangs. Zweckmäßig ist ein Temperatursensor vorgesehen, der die Temperatur des Behälters erfasst und an eine Steuereinrichtung des Injektors übermittelt, wobei die Steuereinrichtung die Temperiereinrichtung mit elektrischem Strom zum Betrieb des Heizelements oder des Kühlelements versorgt und so steuert, dass die Temperatur des Behälters auf der an der Steuereinrichtung eingestellten Solltemperatur gehalten wird.

Zur Vermeidung von Energieverlusten ist es dabei vorteilhaft, wenn die Stromzufuhr an die Temperiereinrichtung von der Steuereinrichtung ausgeschaltet wird, wenn sich die Wärmeübertragungszone der Temperiereinrichtung nicht in Kontakt mit dem Behälter befindet. Hierfür ist zweckmäßig ein Sensor zur Erfassung der Position der Transporteinrichtung bzw. der über die Transporteinrichtung beweglichen Temperiereinrichtung vorgesehen und das vom Sensor erfasste Positionssignal wird an die Steuereinrichtung übertragen, damit die Steuereinrichtung die Stromzufuhr zum Heiz- oder Kühlelement abschalten kann, sobald die Temperiereinrichtung (bzw. die Transporteinrichtung) eine vorgegebene Position erreicht oder verlässt.

In einem bevorzugten Ausführungsbeispiel der Erfindung ist die Temperiereinrichtung gegenüber der Transporteinrichtung um eine senkrecht zur Transportrichtung (Bewegungsrichtung des Schlittens) stehende Achse verschwenkbar. Dies kann bspw. durch eine verschwenkbare Lagerung eines Grundelements der Temperiereinrichtung an einer Vorderseite der Transporteinrichtung, insbesondere an der Vorderseite des Schlittens, realisiert werden. Durch die Verschwenkbarkeit der Temperiereinrichtung gegenüber der Transporteinrichtung kann eine Neigung der Temperiereinrichtung gegenüber einer vertikalen Ebene erfolgen. Dies ermöglicht es, die Temperiereinrichtung auch an Behälter (wie z.B. Flaschen mit einem Flaschenhals) anzuschmiegen, die keine (perfekte) zylindrische Außenfläche aufweisen, und es können Kippbewegungen des Behälters in der Behälteraufnahme ausgeglichen werden.

Zweckmäßig ist das Grundelement im Querschnitt gebogen bzw. konkav gewölbt. An dem Grundelement kann eine thermische Isolierung und darauf das Kühl- oder Heizelement angeordnet sein. Die thermische Isolierung stellt sicher, dass die vom Kühl- oder Heizelement gelieferte Kühl- oder Heizwärme nur (nach vorne) in Richtung des Behälters und nicht (nach hinten) in Richtung des Grundelements strömt, wodurch ein effizienter Wärmeübergang auf den zu temperierenden Behälter erzielt wird. Die thermische Isolierung ist dabei bevorzugt aus einem elastisch verformbaren Material, wie z.B. einem Schaumstoff bzw. einem geschäumten Kunststoff. Dies verbessert die Anschmiegbarkeit der Temperiereinrichtung an eine Außenfläche eines Behälters und erhöht die Wärmeübergangsfläche. Zur Verbesserung und Vergleichmäßigung des Wärmeübergangs kann auf dem Kühl- oder Heizelement zweckmäßig ein Wärmeleitungselement aus einem wärmeleitenden Material, wie z.B. einem Metall, Graphit oder einem wärmeleitfähigen Kunststoff, angeordnet sein, welches an der Außenseite des Behälters anliegt, wenn die Temperiereinrichtung mit einem in der Behälteraufnahme eingesetzten Behälter in thermischem Kontakt steht.

Diese und weitere Vorteile und Merkmale der Erfindung ergeben sich aus dem nachfolgend unter Bezugnahme auf die begleitenden Zeichnungen näher beschriebenen Ausführungsbeispiel. Die Zeichnungen zeigen:
- **Fig. 1:**: Rückansicht eines Injektors zur Injektion eines Fluids in den menschlichen oder tierischen Körper;
- **Fig. 2:**: Seitenansicht des Injektors von Figur 1;
- **Fig. 3:**: Draufsicht auf den Injektor von Figur 1;
- **Fig. 4:**: Schnittdarstellung einer Temperiereinrichtung und einer daran angekoppelten Transporteinrichtung des in den Figuren 1 - 3 gezeigten Injektors in einer rückwärtigen Position;
- **Fig. 5:**: Schnittdarstellung der Temperiereinrichtung und der daran angekoppelten Transporteinrichtung der Figur 4 in der rückwärtigen Position, wobei die Temperiereinrichtung in einer gegenüber der Stellung aus Figur 4 verschwenkten Stellung gezeigt ist;
- **Fig. 6:**: Schnittdarstellung der Temperiereinrichtung und der daran angekoppelten Transporteinrichtung der Figur 4 in einer vorderen Position;

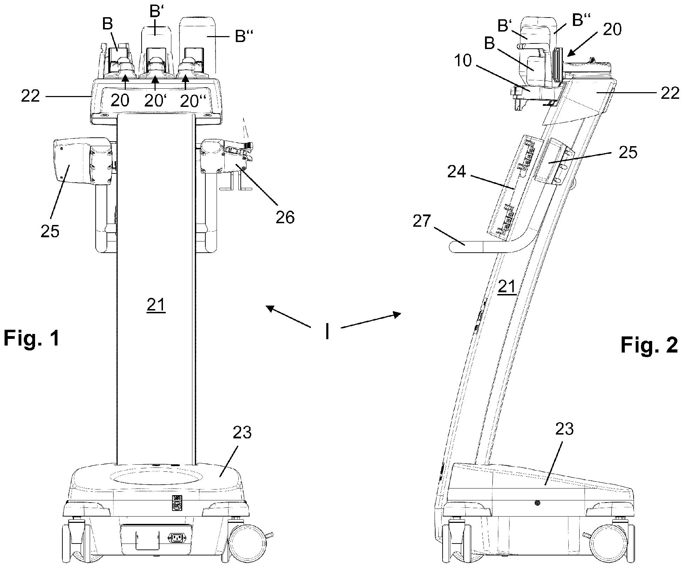

In den Figuren 1 - 3 ist ein Injektor zur Injektion von Kontrastmitteln in den menschlichen oder tierischen Körper dargestellt. Der Injektor I weist ein Rahmengestell mit einem auf Rollen gelagerten Fußteil 23, einem Kopfteil 22 und ein den Fußteil 23 und den Kopfteil 22 verbindendes Schaftteil 21 auf. Im Kopfteil 22 umfasst der Injektor I einen erfindungsgemäßen Behälterhalter, der in dem gezeigten Ausführungsbeispiel drei Behälteraufnahmen 10, 10', 10" zur Aufnahme von Behältern B, B', B" aufweist. Jeder der Behälter B, B', B" ist mit einem Kontrastmittel oder mit einer Spüllösung (beispielsweise einer NaCl-Lösung) gefüllt. Die Behälter B, B', B" sind herausnehmbar in den Behälteraufnahmen 10, 10', 10" eingesetzt. Bei den Behältern (die im Folgenden einheitlich mit B gekennzeichnet sind) handelt es sich beispielsweise um Flaschen unterschiedlicher Form und Größe, die über einen Flaschenhals verfügen und kopfüber (d.h. mit dem Flaschenhals nach unten weisend) in der jeweiligen Behälteraufnahme 10, 10', 10" eingesetzt sind und dort gehalten werden.

Am Schaftteil 21 ist eine Pumpeinrichtung 24 mit einer Schlauchpumpe angeordnet. Weiterhin sind am Schaftteil 21 ein Monitor 25 sowie eine Anschlusseinrichtung 26 befestigt. Die Pumpeinrichtung 24 dient zum Überführen der sich in den Behältern B befindlichen Fluide (Kontrastmittel bzw. Spüllösung) in einen Patientenschlauch. Hierfür wird ein hier nicht dargestellter Patientenschlauch an der Anschlusseinrichtung 26 mit einem (hier ebenfalls nicht dargestellten) Pumpenschlauch verbunden. Der Pumpenschlauch wird über ein Schlauchsystem oder über eine Kassette mit darin angeordneten Fluidkanälen über ein Verzweigungsstück und daran angeordneten Verbindungsschläuchen mit den Behältern B verbunden. Es ist auch möglich, den Patientenschlauch und den Pumpenschlauch in einem Schlauchstück auszubilden und dieses direkt an eine Kassette anzuschließen. Der Pumpenschlauch wird zur Förderung der Fluide von den Behältern B in den Patientenschlauch in die Schlauchpumpe der Pumpeinrichtung 24 eingelegt. Die Behälter B, welche jeweils unterschiedliche Form und Größe aufweisen können, sind mit unterschiedlichen Kontrastmitteln oder mit einer Spüllösung gefüllt und über das Schlauchsystem bzw. die Kassette kann durch Öffnen oder Schließen von Ventilen ausgewählt werden, aus welchem Behälter B das sich darin befindliche Fluid entnommen und mittels der Pumpeinrichtung 24 in den Patientenschlauch gepumpt wird.

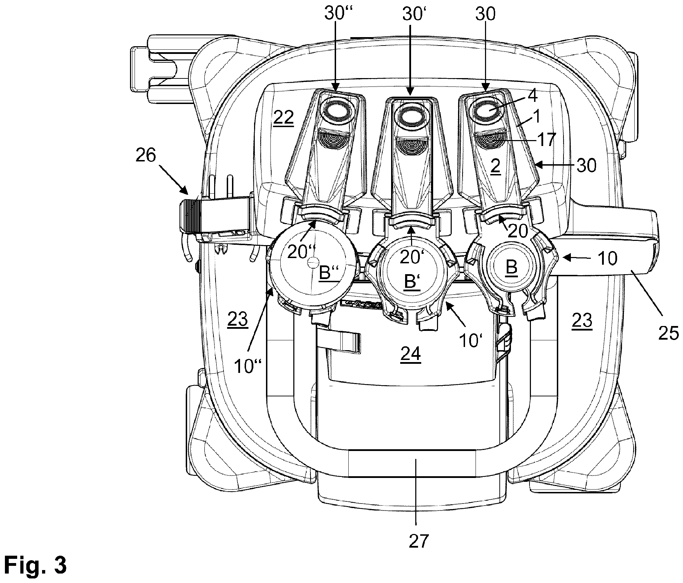

Wie aus Figur 3 ersichtlich, ist an jeder Behälteraufnahme 10, 10', 10" eine zugeordnete Temperiereinrichtung 20, 20', 20" angeordnet. Die Temperiereinrichtungen 20, 20', 20" dienen zur Temperierung der in die Behälteraufnahmen 10, 10', 10" eingesetzten Behälter B, B', B". Jede Temperiereinrichtung 20, 20', 20" ist an eine zugeordnete Transporteinrichtung 30, 30', 30" gekoppelt. Die Transporteinrichtungen 30, 30', 30" dienen zum Heranführen der Temperiereinrichtungen 20, 20', 20" an die in die Behälteraufnahmen 10, 10', 10" eingesetzten Behälter B, B', B".

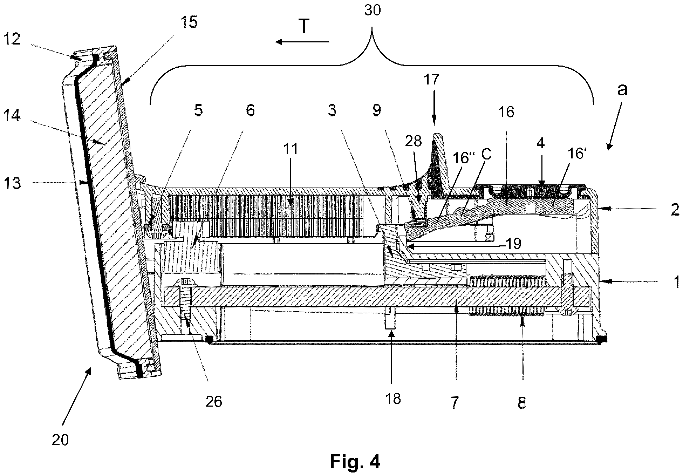

In den Figuren 4 - 6 ist eine Temperiereinrichtung 20 mit einer daran gekoppelten Transporteinrichtung 30 exemplarisch in Längsschnittdarstellungen gezeigt.

Die Temperiereinrichtung 20 ist im Querschnitt gebogen bzw. konkav gewölbt, insbesondere in Form eines Teilzylinders, beispielsweise eines Drittel- oder ViertelZylinders, wie aus Figur 3 ersichtlich. Die Temperiereinrichtung 20 umfasst ein Wärmeleitungselement 12, ein darauf angeordnetes Kühl- oder Heizelement 13, eine darauf angeordnete thermische Isolierung 14 aus einem elastisch verformbaren und nicht wärmeleitenden Material und ein auf der thermischen Isolierung 14 angeordnetes Grundelement 15. Das Wärmeleitungselement 12 ist bspw. als mehrkomponentiges Kunststoff-Spritzgussteil ausgebildet und enthält einen Grundkörper aus einem Hartkunststoff und eine elastische Auflage aus einem Elastomer, wobei die Elastomer-Auflage dem Kühl- oder Heizelement 13 zugewandt und mit diesem thermisch leitend verbunden ist.

An die Temperiereinrichtung 20 ist eine Transporteinrichtung 30 gekoppelt. Die Transporteinrichtung 30 umfasst ein am Injektor I befestigtes Gehäuse 1 und einen gegenüber dem Gehäuse 1 linear entlang einer Bewegungsrichtung T beweglichen, deckelförmigen Schlitten 2. Der Schlitten 2 ist an einer mit dem Gehäuse verbundenen Führung 7 verschiebbar gegen die Rückstellkraft einer Feder 8 gelagert. Hierfür ist an dem Schlitten 2 ein Verbinderteil 3 befestigt und das Verbinderteil 3 ist an der Führung 7 federgelagert und entlang der Führung 7 verschiebbar. An der Unterseite des Verbinderteils 3 ist ein vorstehender Zapfen 18 angeordnet, der nach unten über die Führung 7 vorsteht.

An einer Innenseite des deckelförmigen Schlittens 2 ist eine Zahnstange 11 angeordnet, welche mit einem Zahnrad eines als Rotationsdämpfer ausgebildeten Dämpfungselements 6 kämmt. Das Dämpfungselement 6 ist an dem Gehäuse 1 befestigt. Dadurch wird eine translatorische Bewegung des Schlittens 2 gegenüber dem Gehäuse 1 durch das Dämpfungselement 6 gedämpft.

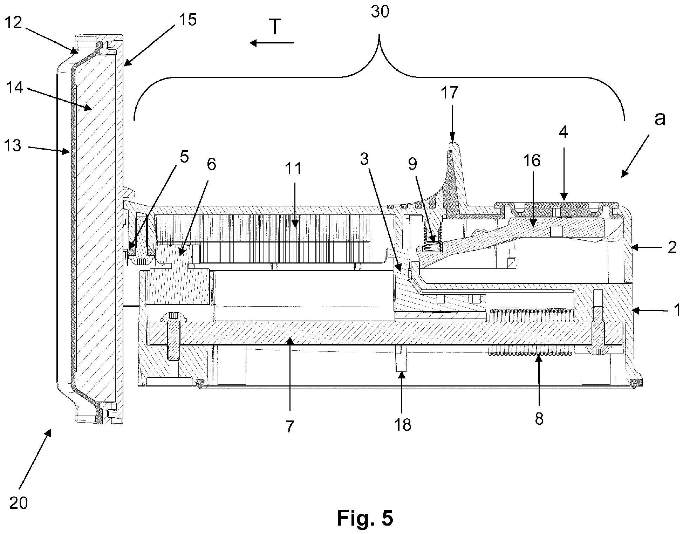

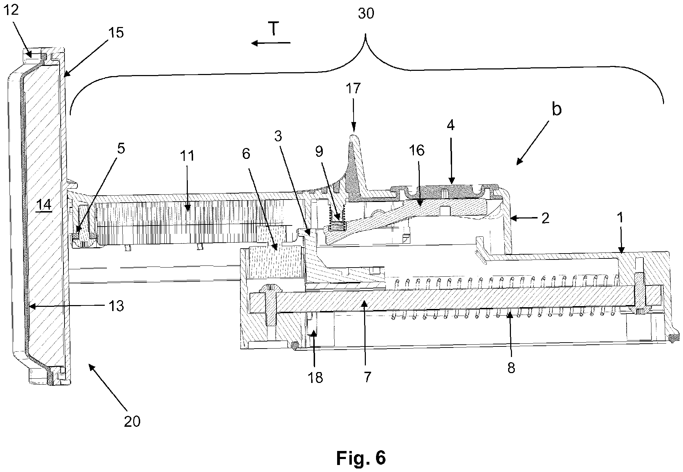

Der Schlitten 2 ist gegenüber dem Gehäuse 1 zwischen einer rückwärtigen Position a, welche in den Figuren 4 und 5 gezeigt ist, und einer vorderen Position b, welche in Figur 6 gezeigt ist, verschiebbar. In der rückwärtigen Position a ist die an der Transporteinrichtung 30 gekoppelte Temperiereinrichtung 20 entfernt von einem in der (in den Figur 4 bis 6 nicht gezeigten) Flaschenaufnahme 10 eingesetzten Behälter B. In der vorderen Position b steht die Temperiereinrichtung 20 mit dem in der Flaschenaufnahme 10 eingesetzten Behälter B in thermischem Kontakt.

Um den Schlitten 2 in seiner rückwärtigen Position a zu fixieren, ist ein Rastmechanismus vorgesehen. Der Rastmechanismus umfasst einen um eine senkrecht zur Bewegungsrichtung T stehende Achse C verschwenkbaren Schwenkhebel 16 sowie eine am Gehäuse 1 angeformte Rastnase 19. Der Schwenkhebel 16 umfasst einen hinteren Flügel 16' und einen vorderen Flügel 16", wobei der vordere Flügel 16" eine Nut aufweist, in die ein am Schlitten 2 angeformter Zapfen 30 mit einer am unteren Ende des Zapfens 30 angeordneten Druckfeder 9 eingreift. Der Schwenkhebel 16 kann um die Achse C verschwenkt werden, indem auf den hinteren Flügel 16' des Schwenkhebels 16 eine Druckkraft ausgeübt wird. Zum Betätigen des Schwenkhebels 16 ist dessen hinterer Flügel 16' mit einem Druckknopf 4 aus einem elastischen Material, beispielsweise einem Elastomer oder Gummimaterial, gekoppelt. Der Druckknopf 4 ist in einer Öffnung in der oberen Wandung des Schlittens 2 eingesetzt und steht mit dem hinteren Flügel 16' des Schwenkhebels 16 in mechanischem Kontakt. Bei (manuellem) Druck auf den Druckknopf 4 wird dieser nach unten in das Innere des Schlittens 2 gepresst und der Druckknopf 4 übt dabei eine Druckkraft auf den hinteren Flügel 16' des Schwenkhebels 16 aus, wodurch dieser um die Achse C entgegen der Rückstellkraft der Druckfeder 9 verschwenkt wird. Durch das Verschwenken des Schwenkhebels 16 löst sich die Vorderkante des vorderen Flügels 16" des Schwenkhebels 16 von der Rastnase 19. Dadurch wird der Rastmechanismus, der den Schlitten 2 in seiner rückwärtigen Position a hält, gelöst und der Schlitten 2 wird durch die Rückstellkraft der Feder 8 linear in Bewegungsrichtung T nach vorne in eine vordere Position b bewegt. Wenn ein Behälter B in die der Temperiereinrichtung 20 zugeordneten Behälteraufnahme 10 eingesetzt ist, wird der Schlitten 2 von der Feder 8 soweit nach vorne geschoben, bis das Wärmeleitungselement 12, welches eine Wärmeübertragungszone bildet, in Kontakt mit der Außenfläche des in die Behälteraufnahme 10 eingesetzten Behälters B kommt. Diese vordere Position b ist in Figur 6 gezeigt. Durch Verschieben des Schlittens 2 in seine vordere Position b wird die Temperiereinrichtung 20 in thermischem Kontakt mit dem Behälter B gebracht.

An der Oberseite des Schlittens 2 ist ein Griffteil 17 angeformt. Das Griffteil 17 dient zum manuellen Zurückschieben des Schlittens 2 in seine rückwärtige Position a. Zum Zurückschieben des Schlittens 2 in seine rückwärtige Position a kann der Schieber 2 manuell durch Ergreifen des Griffteils 17 und Ausüben einer entgegengesetzt zur Bewegungsrichtung T gerichteten Schub- oder Zugkraft und gegen die Rückstellkraft der Feder 8 zurückgeschoben werden, bis der Schlitten 2 in seiner rückwärtigen Position a angelangt ist. Beim Erreichen der rückwärtigen Position a verrastet der Rastmechanismus, indem die vordere Rastkante des vorderen Flügels 16" des Schwenkhebels 16 an der Rastnase 19 des Gehäuses 1 einrastet, wie in den Figuren 4 und 5 gezeigt.

Es ist auch möglich, für das Zurückschieben des Schlittens 2 in seine rückwärtige Position a einen motorischen Antrieb vorzusehen. In diesem Fall kann das Griffteil 17 entfallen.

Zweckmäßig ist ein in den Zeichnungen nicht dargestellter Sensor zur Erfassung der Position der Transporteinrichtung 30 vorgesehen. Der Sensor zur Erfassung der Position der Transporteinrichtung 30 kann beispielsweise als optischer Sensor ausgebildet sein, der die Position des an der Unterseite des Verbindungsteils 3 angeordneten Zapfens 18 erfasst. Bei dem Sensor zur Erfassung der Position der Transporteinrichtung 30 kann es sich jedoch auch um einen Positionsschalter handeln, wie z.B. berührungssensitive Endschalter oder induktive oder kapazitive Positionssensoren. Der Sensor zur Erfassung der Position der Transporteinrichtung 30 ist mit einer Steuerung gekoppelt, welche die Temperiereinrichtung 20 mit elektrischem Strom zum Betrieb des Kühl- oder Heizelements 13 versorgt. Die Steuerung ist dabei so eingerichtet, dass die Stromversorgung des Kühl- oder Heizelements 13 abgeschaltet wird, sobald sich der Schlitten 2 in seiner rückwärtigen Position a befindet. Sobald der Schlitten 2 seine rückwärtige Position a verlässt und in eine vordere Position b gebracht wird, schaltet die Steuerung die Stromversorgung des Kühl- oder Heizelements 13 an, so dass die Temperiereinrichtung 20 zur Temperierung eines in die Behälteraufnahme 10 eingesetzten Behälters B aktiviert wird.

In der Behälteraufnahme 10 ist zweckmäßig ein Temperatursensor zur Erfassung der Temperatur eines in die Behälteraufnahme 10 eingesetzten Behälters B vorgesehen. Der Temperatursensor ist ebenfalls mit der Steuerung gekoppelt und übermittelt die erfasste Temperatur des Behälters B an die Steuerung, welche die Stromzufuhr zu dem Kühl- oder Heizelement 13 entsprechend der vom Temperatursensor erfassten Temperatur so regelt, dass die Temperatur des Behälters B auf eine vorgegebene Soll-Temperatur gebracht und während eines Injektionsvorgangs auf dieser Soll-Temperatur gehalten wird.

Wie aus einem Vergleich der Figuren 4 und 5 ersichtlich wird, kann die Temperiereinrichtung 20 gegenüber einer vertikalen Ebene verschwenkt werden. Hierfür ist die Temperiereinrichtung 20 über ein Gelenk 5 an dem Schlitten 2 um eine senkrecht zur Bewegungsrichtung T stehende Achse verschwenkbar angelenkt. Das Gelenk 5 ermöglicht ein Verschwenken der Temperatureinrichtung 20, bevorzugt innerhalb eines begrenzten Winkelbereichs von bspw. ± 5° bis ± 20° um die in Figur 5 gezeigte vertikale Grundstellung der Temperiereinrichtung 20.

In einer zweckmäßigen Ausführungsform ist das Gehäuse 1 der Transporteinrichtung 30 aus einem durchsichtigen oder transluzenten Kunststoff gefertigt und im Gehäuse 1 ist eine Lichtquelle, beispielsweise eine LED, angeordnet. Die Lichtquelle wird dabei von der Steuerung gesteuert, um Funktionszustände des Injektors I und/oder der Temperiereinrichtung anzuzeigen. So kann beispielsweise über unterschiedliche Leuchtfarben der Lichtquelle angezeigt werden, ob sich in der Behälteraufnahme 10, die der Temperiereinrichtung 20 und der zugehörigen Transporteinrichtung 30 zugeordnet ist, ein Behälter B befindet und/oder ob die Temperiereinrichtung 20 aktiviert ist. Eine weitere Lichtquelle, insbesondere in Form einer LED, kann auch an der Temperiereinrichtung 20 angebracht sein, bspw. am Grundelement 15 oder am Kühl- oder Heizelement 13, um Funktionszustände der Temperiereinrichtung 20 anzuzeigen.

## Patentansprüche

1. Behälterhalter für einen Injektor (I) zur Injektion eines Fluids in den menschlichen oder tierischen Körper, mit wenigstens einer Behälteraufnahme (10) zur Aufnahme eines mit dem Fluid gefüllten Behälters (B) und einer Temperiereinrichtung (20) zur Temperierung des Behälter (B) auf eine vorgegebene Temperatur, **dadurch gekennzeichnet, dass** die Temperiereinrichtung (20) an eine Transporteinrichtung (30) gekoppelt ist, mit der die Temperiereinrichtung (20) mit dem in der Behälteraufnahme (10) eingesetzten Behälter (B) in und außer thermischem Kontakt bringbar ist.

2. Behälterhalter nach Anspruch 1, **dadurch gekennzeichnet, dass** die Temperiereinrichtung (20) ein regelbares Heizelement (13) oder Kühlelement umfasst.

3. Behälterhalter nach Anspruch 1 oder 2, **dadurch gekennzeichnet, dass** die Temperiereinrichtung (20) ein Wärmeleitungselement (12) umfasst, welches an einer Außenseite des Behälters (B) anliegt, wenn die Temperiereinrichtung (20) mit dem in der Behälteraufnahme (10) eingesetzten Behälter (B) in thermischem Kontakt steht.

4. Behälterhalter nach einem der voranstehenden Ansprüche, **dadurch gekennzeichnet, dass** die Temperiereinrichtung (20) mittels der Transporteinrichtung (30) zwischen einer rückwärtigen Position (a) und mindestens einer vorderen Position (b) verschiebbar ist, wobei die Temperiereinrichtung (20) in der rückwärtigen Position (a) entfernt von dem in der Behälteraufnahme (10) eingesetzten Behälter (B) ist und in der vorderen Position (b) in thermischem Kontakt mit dem Behälter (B) steht.

5. Behälterhalter nach Anspruch 4, **gekennzeichnet durch** einen Rastmechanismus, der die Transporteinrichtung (30) in der rückwärtigen Position (a) und/oder in der vorderen Position (b) fixiert.

6. Behälterhalter nach Anspruch 5, **dadurch gekennzeichnet, dass** der Rastmechanismus einen manuell betätigbaren Druckknopf (4) umfasst, der bei Betätigung eine Verrastung der Transporteinrichtung (30) in der rückwärtigen Position (a) und/oder in der vorderen Position (b) frei gibt.

7. Behälterhalter nach einem der voranstehenden Ansprüche, **dadurch gekennzeichnet, dass** die Transporteinrichtung (30) ein am Injektor (I) fixierbares Gehäuse (1) und einen gegenüber dem Gehäuse (1) beweglichen Schlitten (2) umfasst.

8. Behälterhalter nach Anspruch 7, **dadurch gekennzeichnet, dass** der Schlitten (2) in oder an dem Gehäuse (1) federgelagert ist und/oder dass der Schlitten (2) verschiebbar an einer Führung (7) gelagert ist.

9. Behälterhalter nach einem der voranstehenden Ansprüche, **dadurch gekennzeichnet, dass** die Temperiereinrichtung (20) ein Grundelement (15) umfasst, welches an einer Vorderseite der Transporteinrichtung (30), insbesondere an der Vorderseite des Schlittens (2), verschwenkbar angelenkt ist.

10. Behälterhalter nach einem der Ansprüche 7 bis 9, **dadurch gekennzeichnet, dass** die Transporteinrichtung (30) ein Dämpfungselement (6), aufweist, welches die Bewegung des Schlittens (2) dämpft.

11. Behälterhalter nach einem der Ansprüche 9 oder 10, **dadurch gekennzeichnet, dass** an dem Grundelement (15) der Temperiereinrichtung (20) ein Kühl- oder Heizelement (13) und zwischen dem Grundelement (15) und dem Kühl- oder Heizelement (13) eine thermische Isolierung (14) angeordnet ist, wobei die thermische Isolierung (14) bevorzugt aus einem elastisch verformbaren Material ist.

12. Behälterhalter nach einem der Ansprüche 7 bis 11, **dadurch gekennzeichnet, dass** der Schlitten (2) ein angeformtes Griffteil (17) aufweist, mit dem der bewegliche Schlitten (2) manuell von einer vorderen Position (b), in der sich die Temperiereinrichtung (20) in thermischem Kontakt mit dem Behälter (B) befindet, in eine rückwärtige Position (a), in der sich die Temperiereinrichtung (20) im Abstand zu dem Behälter (B) befindet, verschiebbar ist.

13. Behälterhalter nach einem der voranstehenden Ansprüche, **gekennzeichnet durch** einen Sensor zur Erfassung der Position der Temperiereinrichtung (20) und/oder der Transporteinrichtung (30), wobei der Sensor und die Temperiereinrichtung (20) mit einer Steuerung gekoppelt sind, welche die Temperiereinrichtung (20) mit elektrischem Strom zum Betrieb des Kühl- oder Heizelements (13) versorgt, sobald die Temperiereinrichtung (20) eine vorgegebene Position erreicht oder verlässt.

14. Behälterhalter nach einem der voranstehenden Ansprüche, **dadurch gekennzeichnet, dass** die Transporteinrichtung (30) eine Feder (8) enthält und die Temperiereinrichtung (20) von der Rückstellkraft der Feder (8) in Richtung eines in der Behälteraufnahme (10) eingesetzten Behälters (B) bewegt wird, um die Temperiereinrichtung (20) in thermischem Kontakt mit dem Behälter (B) zu bringen.

15. Verfahren zur Temperierung eines mit einem Injektionsfluid gefüllten und in einer Behälteraufnahme (10) eines Injektors (I) eingesetzten Behälters (B), mit folgenden Schritten:
- Einsetzen des Behälters (B) in eine Behälteraufnahme (10) eines Injektors (I),
- Herstellen eines thermischen Kontakts zwischen einer Temperiereinrichtung (20) und dem in der Behälteraufnahme (10) eingesetzten Behälter (B) durch Heranschieben der Temperiereinrichtung (20) an den Behälter mittels einer an der Behälteraufnahme (10) angeordneten Transporteinrichtung (30), wobei das Heranschieben der Temperiereinrichtung (20) an den Behälter (B) bevorzugt durch die Rückstellkraft einer Feder (8) oder durch die Spannkraft eines Vorspannelements bewirkt wird,
- Aktivieren der Temperiereinrichtung (20) zur Temperierung des Behälters (B) auf eine vorgegebene Temperatur.
